# EUROPEAN PATENT APPLICATION

(11) **EP 3 326 624 A1**
(43) Date of publication of application: **30.05.2018**
(21) Application number: 15756941.9
(22) Date of filing: 22.07.2015
(51) Int. Cl.: A61K 31/137, A61P 35/00, A61K 45/06, G01N 33/50, A61K 39/395

(54) **IMMUNOTHERAPY FOR CASR-EXPRESSING CANCER (E.G. NEUROBLASTOMA)**

(71) Applicant: Hospital Sant Joan de Deu, 08950 Esplugues de Llobregat Barcelona (ES); Fundació Cellex, 08010 Barcelona (ES)
(72) Inventor: DE TORRES GÓMEZ-PALLETE, Carmen, E-08950 Esplugues de Llobregat (Barcelona) (ES)
(74) Representative: Herrero & Asociados, S.L.
(86) International application number: PCT/ES2015/070561
(87) International publication number: WO 2017/013276

(57) **Abstract**

The present invention refers to the field of cancer. In particular, it refers to methods for increasing susceptibility of cancer cells to immunotherapeutic agents and methods for assessing the effectiveness of a cancer treatment.

## Description

### Field of the invention

The present invention refers to the field of cancer and, in particular, to cancer immunotherapy.

### Background of the invention

The calcium-sensing receptor (CaSR) is a family C G-protein coupled receptor (GPCR) that senses minimal plasmatic fluctuations of Ca²⁺ and regulates the secretion of parathyroid hormone accordingly to maintain extracellular concentrations of this cation within a very narrow range. The CaSR is also expressed in many organs and tissues not involved in Ca²⁺ homeostasis. This GPCR is able to detect variations of other ions, polyamines and L-aminoacids, among other substances, and to couple to several G-proteins (G_{q/11}, G_{12/13}, Gᵢ and Gₛ), therefore activating different downstream signaling pathways. This versatile mechanism allows the CaSR to play a wide variety of cell type-specific roles in physiologic and in pathological conditions, including cancer.

CaSR activators can be divided in orthosteric ligands or direct CaSR agonists (also known as type 1 agonists or type I calcimimetics) and allosteric activators of CaSR (also known as type 2 agonists or type II calcimimetics). Type I calcimimetics such as Ca²⁺, Mg²⁺, Gd³⁺, neomycin, L-amino acids, activate the CaSR by means of direct interaction with the extracellular domain thereof. In contrast, allosteric activators, such as Cinacalcet, do not activate the receptor *per se* but rather modify its three-dimensional structure, such that it becomes more sensitive to the calcium stimulus. Cinacalcet has been approved to treat severe hyperparathyroidism (HPT) in patients being dialyzed for chronic kidney disease, hypercalcemia in the context of parathyroid carcinoma, and severe hypercalcemia in patients with primary HPT when surgical treatment is not possible. Moreover, WO 2013144397 describes the use of Cinacalcet in the treatment of neuroblastic tumors.

Neuroblastic tumors encompass a heterogeneous group of developmental malignancies that arise from peripheral nervous system precursor cells, and include the neuroblastomas, ganglioneuroblastomas and ganglioneuromas. Although some of them undergo spontaneous regression and others progress only as local-regional tumors, around 50% are present as metastasic neuroblastomas. The outcome of most patients in the two former groups is excellent, even without cytotoxic therapy. However, overall survival rates of patients diagnosed with metastatic disease remain below 40% despite the current multimodal approach that includes chemotherapy, surgery, radiotherapy, autologous bone marrow transplant, differentiating agents and immunotherapy. Moreover, around 60% of metastatic neuroblastomas in patients older than 18 months ultimately relapse, and, to date, there is no treatment known to be curative for these patients.

An immunotherapeutic agent targeting a tumor-associated antigen, the disialoganglioside GD2, has become part of the standard of care for high-risk neuroblastomas (metastatic cases and refractory local-regional tumors). Immunotherapeutic agent with ch14.18 (a chimeric human-murine anti-GD2 monoclonal antibody) combined withgranulocyte-macrophage colony-stimulating factor and interleukin-2 has been reported to be associated with a significantly improved outcome as compared with standard therapy in patients with high-risk neuroblastoma. However, GD2 is also expressed in neurons, skin melanocytes, and peripheral sensory nerve fibers. This pattern of expression contributes to the important treatment-related toxic effects of this immunotherapeutic agent regimen, including severe pain, hypotension, capillary leak syndrome, and hypersensitivity reactions.

Moreover, a limitation of molecularly targeted therapies in cancer is the lack of markers that allow to evaluate tumor response to the administered drug, particularly markers that can be detected using non-invasive techniques. Many of these drugs do not cause dramatic shrinkage of tumor volume that can be monitored by imaging techniques, making it difficult to assess the effects of the drug unless biopsies are repeatedly performed.

Therefore, there is in the state of the art the need to increase the susceptibility of cancer cells to immunotherapeutic agent without affecting normal tissues, to provide new cancer therapies, and to provide surrogate markers which allow to evaluate the response of a patient diagnosed with a cancer to the therapy administered for the treatment of said cancer.

### Summary of the invention

In a first aspect, the present invention refers to a CaSR activator agent for use in increasing the susceptibility of a subject with a CaSR-expressing cancer to an immunotherapeutic agent based on CTAs.

In a second aspect, the present invention refers to a CaSR activator agent in combination with an immunotherapeutic agent based on CTAs for use in the treatment of a CaSR-expressing cancer.

In a third aspect, the present invention refers to a kit comprising a CaSR activator agent and an immunotherapeutic agent based on CTAs as combination for simultaneous, separate or successive administration.

In a fourth aspect, the present invention refers to an *in vitro* method for assessing the effectiveness of a therapy administered to a subject with a CaSR expressing cancer, comprising the following steps:
a) quantifying the expression level of a CTA gene in a biological sample from said subject before and after the therapy, and
b) comparing the expression level of the CTA gene in the biological samples,
wherein the therapy administered comprises a CaSR activator agent and wherein an increase of the expression level of the CTA gene after the therapy with respect to the expression level of said CTA gene before the therapy, is indicative that the therapy is effective.

In a fifth aspect, the present invention refers to a kit for assessing the effectiveness of a therapy administered to a subject with a CaSR expressing cancer comprising appropriate means to measure the expression level of a CTA gene of the SSX family and a CTA gene of the GAGE family, and a suitable packaging.

In a sixth aspect, the present invention refers to the use of a CTA gene as a surrogate marker of tumor response of a CaSR-expressing cancer to treatment with a CaSR activator agent.

Other objects, features, advantages and aspects of the present application will become apparent to those skilled in the art from the following description and appended claims.

### Brief description of the drawing

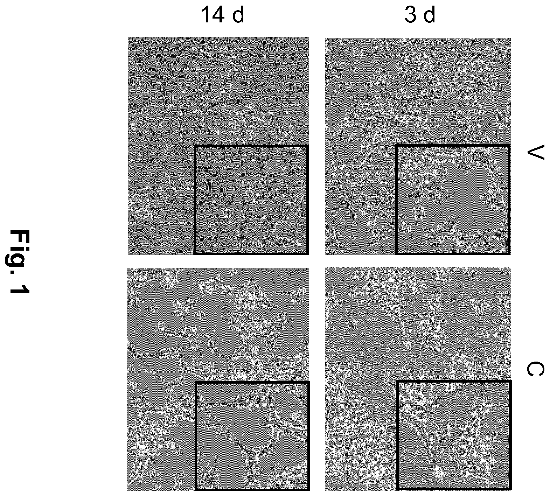

**Figure 1** is a micrograph showing the morphological assesment of LA-N-1 cells exposed to 1 µM Cinacalcet (C) or DMSO (vehicle (V)) for 3 days (3d) and 14 days (14d).

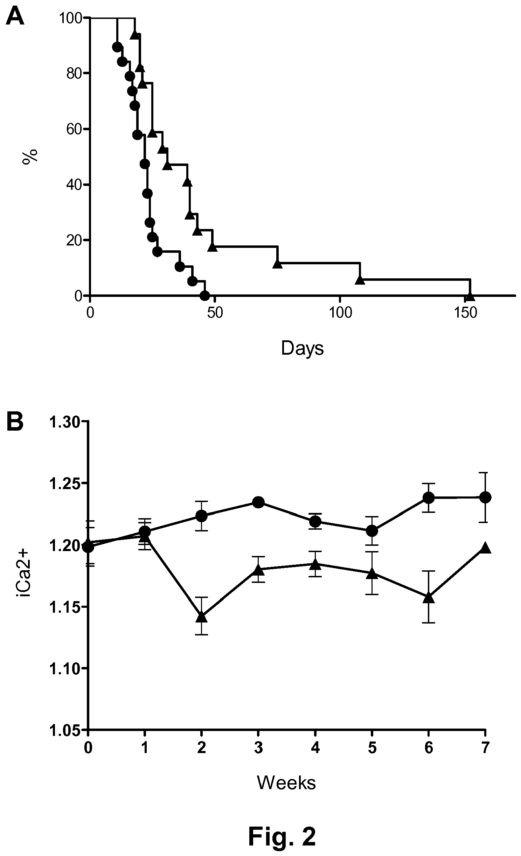

**Figure 2** comprises graphs showing **(A)** Effect of Cinacalcet treatment on survival (%) in an *in vivo* neuroblastoma model treated with Cinacalcet (triangle) or vehicle (circle) over time (days); **(B)** Plasmatic ionic Ca²⁺ concentrations (mmol/L) in mice receiving Cinacalcet (triangle) or vehicle (circle) over time (weeks).

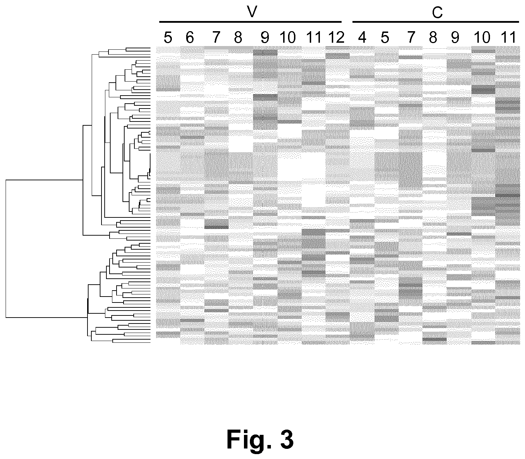

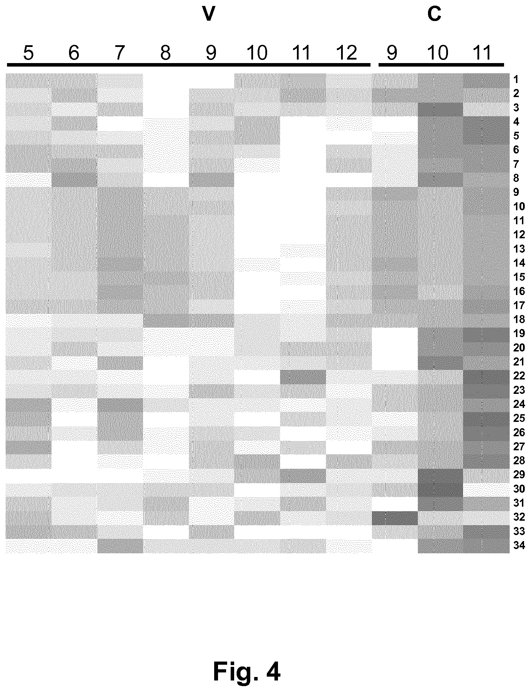

**Figure 3** and **Figure 4** are a visual representation of the expression profile of various genes (93 probe sets in Fig. 3, and 34 in Fig. 4) for different speciments treated with Cinacalcet (C) or vehicle (V). In the heatmaps upregulated genes are represented in red which in greyscale tends to be presented by darker shades. Lighter shades in greyscale tend to present green on the heatmap (genes which are not upregulated).

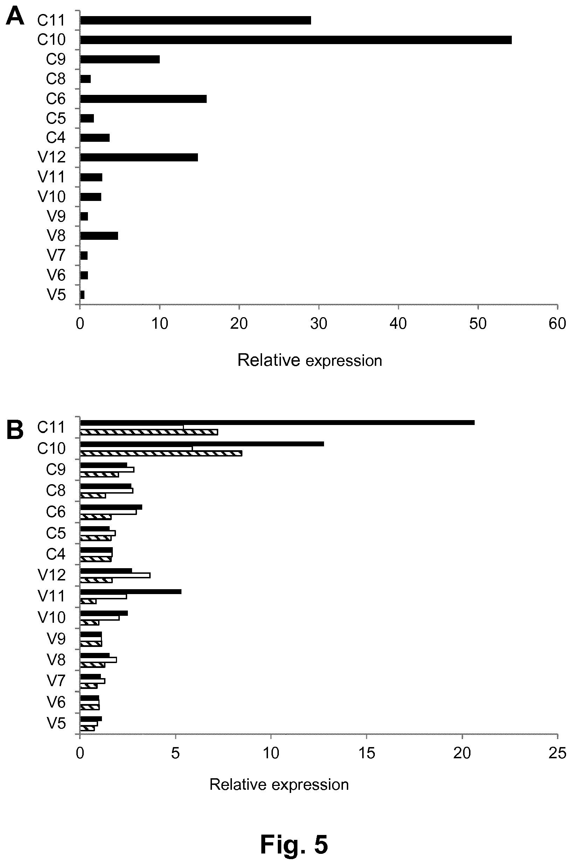

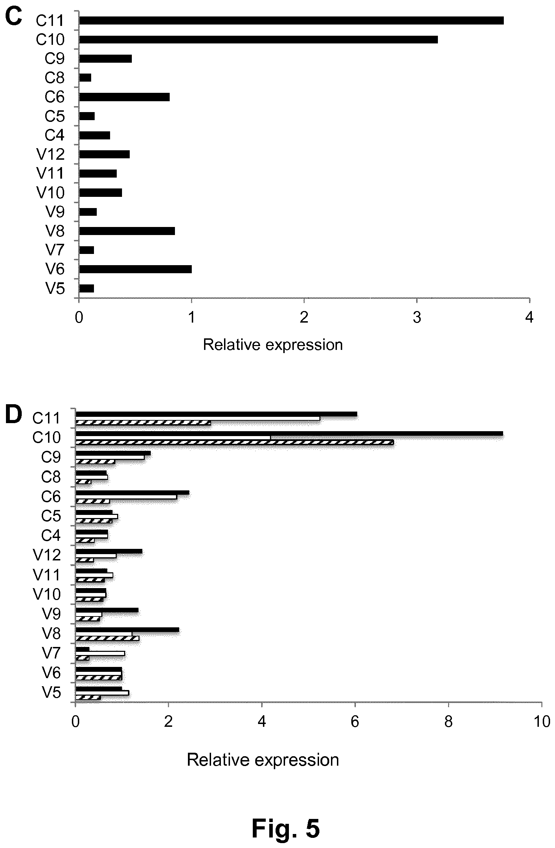

**Figure 5** comprises graphs showing **(A)** Relative mRNA expression of *CTCFL* as determined by reverse transcription quantitative PCR (RT-qPCR) in specimens treated with Cinacalcet (C) or vehicle (V). **(B)** Relative mRNA expression of *SSX4*/*4B* (black), *MAGE A3* (white), *MAGE A2* (striped) in the same model. **(C)** Relative mRNA expression of *NTRK3* in the same model. **(D)** Relative mRNA expression of *ADCY8* (black), *PRKCA* (white), *RYR2* (striped) in the same model.

### Detailed description of the invention

It must be noted that as used in the present application, the singular forms "a", "an" and "the" include their correspondent plurals unless the context clearly dictates otherwise.
Current challenges in applying immunotherapy in the treatment of cancer include the identification of suitable tumor antigens. Ideal targets for immunotherapy would be those antigens which are immunogenic and which are specifically expressed in tumor cells.
Cancer-testis antigens (CTAs) encompass a large family of more than 240 tumor-associated antigens, details of which can be obtained from http://www.cta.lncc.br. CTAs can be divided in those that are encoded by genes mapping on the X chromosome, the X-CTA genes, and those that are not encoded by genes mapping on the X chromosome, the non-X-CTA genes. X-CTAs include multigene families organized in clusters along the X chromosome and include, among others, the *MAGE, GAGE, SSX* and *NY-ESO* gene families. Non-X-CTAs are distributed throughout the genome, are mostly single-copy genes and include, among others, *BORIS* (*CTCFL*) gene.
CTAs are expressed in tumors of different histogenesis, but not in normal tissues, except for testis, fetal ovary and placenta, which are not accesible to the immune system. Moreover, CTA have a strong *in vivo* immunogenicity. Thus, their tumor-restricted pattern of expression and strong immunogenicity make CTAs ideal targets for different tumor-specific immunotherapeutic approaches.
Although CTAs are immunogenic tumoral antigens, the remarkable inter- and intratumoral heterogeneous expresion of CTAs limits the eligibility of cancer patients to treatment; and/or impairs immune recognition of neoplastic cells thus reducing the efficacy of immunotherapies based on CTAs.
Surprisingly, the inventors have developed an approach to modulate CTAs expression in neoplastic cells. In particular, the inventors have found that a CaSR activator agent increases the expression of CTA genes *in vitro* and *in vivo,* and, interestingly, most of the overexpressed genes are X-CTA genes, which are the most immunogenic group of CTAs. Thus, the inventors are providing an approach to increase the expression level of immunogenic targets, which are essentially tumor-specific, making cancer cells more susceptible to immunotherapeutic agents based on CTAs and enhancing the efficacy of a treatment with immunotherapeutic agents based on CTAs. Surprisingly, the inventors have also found that such increase in CTAs expression can be used as surrogate marker of cancer response to a treatment with a CaSR activator agent. Thus, this increase in CTAs expression provides new uses, methods and products, which are the object of the present invention.
As shown in the Examples, Cinacalcet, a CaSR activator agent, is able to increase the expression of CTA genes *in vitro* and *in vivo.* CTAs are genes almost exclusively expressed in cancer, and thus increasing their expression level makes tumor cells, and therefore a subject with such cells, more susceptible to immunotherapeutic agents specifically directed against said CTAs. Thus, in a first aspect, the present invention refers to a CaSR activator agent for use in increasing the susceptibility of a subject with a CaSR-expressing cancer to an immunotherapeutic agent based on CTAs.

The CaSR activator agent increases the expression level of at least one CTA gene, thus, the first aspect of the invention also refers to a CaSR activator agent for use in increasing the susceptibility of a subject with a CaSR-expressing cancer to an immunotherapeutic agent based on CTAs, wherein the CaSR activator agent increases the expression level of at least one CTA gene.
The first aspect of the present invention also refers to the use of a CaSR activator agent for increasing the susceptibility in a subject with a CaSR-expressing cancer to an immunotherapeutic agent based on CTAs.
The first aspect of the present invention also refers to the use of a CaSR activator agent as enhancer of the susceptibility of a subject with a CaSR-expressing cancer to an immunotherapeutic agent based on CTAs.
The first aspect of the present invention further refers to a method for increasing the susceptibility to an immunotherapeutic agent based on CTAs of a subject with a CaSR-expressing cancer, comprising the administration of a CaSR activator agent to the subject to increase the expression level of a CTA gene.
In the context of the present invention, the term "CaSR activator agent" refers to direct CaSR agonists and to allosteric activators of CaSR. In a preferred embodiment of the first aspect of the invention according to any of the paragraphs above, the CaSR activator agent is a CaSR allosteric activator. There are multiple allosteric activators of the CaSR, among others, cinacalcet, NPS-R568, calindol, calcimimetic B and AC-265347. Thus, in a more preferred embodiment, the CaSR activator agent is selected from Cinacalcet, NPS-R568, calindol, calcimimetic B and AC-265347 and combinations thereof, and in an even more preferred embodiment the CaSR activator agent is Cinacalcet.
These compounds are well known by the skilled in the art and can be synthesed using standard techniques, as for example the ones described in EP 1913941 a1, Nemeth et al. (Calcimimetics with potent and selective activity on the parathyroid calcium receptor". Proc Natl Acad Sci USA. 1998 31;95(7):4040-5). Moreover, these compounds are commercially available from , for example, the following commertial branches Cinacalcet (Amgen, Selleck Chemicals), NPS-R-568 (NPS Pharmaceuticals/Shire), Calcimimetic B (Amgen), AC-265347 (ACADIA Pharmaceuticals, Sigma-Aldrich), Calindol (Toronto Research Chemicals).
In the context of the present invention the term "immunotherapeutic agent based on CTAs", refers to an immunotherapeutic agent specific for at least a CTA, i.e. at least a CTA is the target molecule for the immunotherapeutic agent. CTA refers to a CTA gene, a fragment thereof, a CTA protein, a fragment thereof (CTA peptide), or a combination thereof. Immunotherapeutic agent refers to an agent capable of stimulating an immune response. Said immune response results in the treatment, amelioration and/or retardation of the progression of a disease. In particular, immunotherapeutic agents are, amongst others, vaccines using CTA peptides as vaccinating agents; dendritic cells vaccines pulsed with these peptides; and T-cells expressing native or engineered receptors recognizing these peptides.
In the context of the present invention the term "susceptibility to an immunotherapeutic agent based on CTAs" refers to sensitivity to an immunotherapeutic agent based on CTAs. With an increased in the susceptibility or sensitivity of CaSR-expressing cancer cells to an immunotherapeutic agent based on CTAs a better efficacy of the treatment with such immunotherapeutic agent based on CTAs of a subject with a CaSR-expressing cancer agent is expected.
Likewise, the term "as enhancer of the susceptibility to an immunotherapeutic agent based on CTAs" refers to an agent that enhances the susceptibility or sensitivity of an immunotherapeutic agent based on CTAs.
The term "CaSR-expressing cancer" refers to a cancer in which the CaSR is expressed in the plasma membrane of the cancer cells. In a particular embodiment of the first aspect of the invention according to any of the paragraphs above, the CaSR-expressing cancer is selected from parathyroid adenoma and carcinoma, colon cancer, breast cancer, prostate cancer, glioma, Leydig cell cancer, ovarian cancer, neuroblastomas, ganglioneuroblastomas, ganglioneuromas, bone metastases (in particular bone metastases of renal cancer carcinoma, breast cancer or prostate cancers), and combinations thereof. In a preferred embodiment, the CaSR-expressing cancer is selected from neuroblastomas, ganglioneuroblastomas, ganglioneuromas and combinations thereof; and more preferably the CaSR-expressing cancer is neuroblastoma. As shown in the literature, all these cancers are CaSR-expressing cancers.

As shown in Examples 1, 3 and 4, the expression level of CTAs of the SSX and GAGE families is increased upon administration of Cinacalcet *in vitro* and *in vivo,* and it is detected by microarrays and by RT-qPCR. Thus, in a particular embodiment of the first aspect of the invention according to any of the paragraphs above, the susceptibility to an immunotherapeutic agent based on a CTA of the SSX family is increased, in another embodiment the susceptibility to an immunotherapeutic agent based on a CTA of the GAGE family is increased, and in another embodiment the susceptibility to an immunotherapeutic agent based on a CTA of the SSX family and to an immunotherapeutic agent based on a CTA of the GAGE family is increased. In a preferred embodiment, the CTA of the SSX family is selected from SSX 4 and/or SSX 4B; and the CTA of the GAGE family is selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 121, GAGE 12J, GAGE 13 and combinations thereof. Preferably the CTAs of the GAGE family are GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. In a yet more preferred embodiment, the susceptibility is increased to immunotherapeutic agents specific for SSX 4 and/or SSX 4B; and, for GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. And more preferably, the susceptibility to immunotherapeutic agents specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H and GAGE 12J is increased.
As shown in Example 4, the expression level of CTAs of the MAGE families is increased upon administration of Cinacalcet. Thus, in another preferred embodiment of the first aspect of the invention according to the embodiments of the previous paragraph, the susceptibility to an immunotherapeutic agent based on a CTA of the MAGE family is increased. Preferably the CTA of the MAGE family is MAGE A2 and/or MAGE A3, more preferably MAGE A2, and even more preferably MAGE A2 and MAGE A3.
In another preferred embodiment of the first aspect of the invention according to any of the embodiments of the two previous paragraphs, the susceptibility to an immunotherapeutic agent based on a CTA of the non-X-CTA family is increased, preferably based on CTCFL.

More preferably, the susceptibility to immunotherapeutic agents specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3, and CTCFL, is increased.
Since expression of CTAs has been described to show remarkable inter- and intra-tumoral heterogenous distribution (i.e. different tumors show a different CTA-expression pattern, and different cells within the same tumor show different patterns of expression of these antigens), the best immunotherapeutic agent will comprise several immunotherapeutic agent targeting several CTAs simultaneously, as the ones described in the above paragraphs. This kind of multi-target approach has also been shown to be the most effective in the context of other immunotherapies such as dendritic cell vaccines for treatment of glioblastoma, where antigens to be targeted also show high inter- and intra-tumoral heterogeneous expression.

Since a CaSR activator agent increases the expression level of at least one CTA in CaSR expressing cancer cells, making said cells more susceptible to at least an immunotherapeutic agent based on said CTA, the present invention also refers to a CaSR activator agent and an immunotherapeutic agent based on CTAs, as a combination therapy, for use in the treatment of a CaSR expressing cancer. Thus, in a second aspect, the present invention refers to a CaSR activator agent in combination with an immunotherapeutic agent based on CTAs for use in the treatment of a CaSR-expressing cancer. The second aspect of the present invention also refers to the use of a CaSR activator agent in combination with an immunotherapeutic agent based on CTAs for the manufacture of a medicament for the treatment of a CaSR-expressing cancer.
In a particular embodiment of the second aspect of the invention, the CaSR activator agent is an allosteric activator of CaSR, in a preferred embodiment the CaSR activator agent is selected from Cinacalcet, NPS-R568, calindol, calcimimetic B, AC-265347 and combinations thereof, and more preferably the CaSR activator agent is Cinacalcet.
In another particular embodiment of the second aspect of the invention as defined in any of the paragraphs above, the CaSR-expressing cancer is selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof. In a preferred embodiment, the CaSR-expressing cancer is selected from neuroblastomas, ganglioneuroblastomas, ganglioneuromas and combinations thereof; and more preferably is neuroblastoma.
As used herein, the term "in combination" does not restrict the order in which the CaSR activator agent and the immunotherapeutic agent based on CTAs are administered to a subject. A CaSR activator agent can be administered to a subject prior to (e.g., at least 3 days, 1 week, 2 weeks, 3 weeks, 4 weeks, 5 weeks, 6 weeks, 8 weeks, or 12 weeks before), concomitantly with, or subsequent (e.g., at least 3 days, 1 week, 2 weeks, 3 weeks, 4 weeks, 5 weeks, 6 weeks, 8 weeks, or 12 weeks after) to the administration of the immunotherapeutic agent based on CTAs. The administration of the CaSR activator agent and the immunotherapeutic agent based on CTAs may be by the same or different routes.
In a particular embodiment of the second aspect of the invention according to any of the paragraphs above, the CaSR activator agent is administered prior and/or simultaneously to the immunotherapeutic agent based on CTAs. Preferably, the CaSR activator agent is administered prior to the immunotherapeutic agent based on CTAs, so the expression of CTAs is increased before the administration of the immunotherapeutic agent based on CTAs, and subsequently the immunotherapeutic agent is administered. Preferably the administration of the CaSR activator agent is maintained even after administering the immunotherapeutic agent based on CTAs, so the effects of the CaSR activator agent are maintained while administering the immunotherapeutic agent based on CTAs.
In a particular embodiment of the second aspect of the invention according to any one of the embodiments of the paragraphs above, the immunotherapeutic agent based on CTAs comprises an immunotherapeutic agent specific for a CTA of the SSX family and/or an immunotherapeutic agent specific for a CTA of the GAGE family.
In a preferred embodiment of the second aspect according to the paragraph above, the immunotherapeutic agent based on CTAs comprises an immunotherapeutic agent specific for:
- a CTA of the SSX family selected from SSX 4 and/or SSX 4B, and/or
- a CTA of the GAGE family selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 121, GAGE 12J, GAGE 13 and combinations thereof. In particular, 1, 2, 3, 4, 5, 6, 7, 8, 9, 10, 11, 12, 13, 14, 15, 16, 17, 18, 19, 20, 21 or 22 of the previously mentioned GAGEs. Preferably GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. In a yet more preferred embodiment, the immunotherapeutic agent based on CTAs comprises immunotherapeutic agents specific for SSX 4 and/or SSX 4B; and, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. And even more preferably specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H and GAGE 12J.
Since the expression level of a CTA gene of the MAGE family is also increased (see Examples 1 and 4), in a particular embodiment of the second aspect of the invention according to any of the embodiments of the two previous paragraphs, the immunotherapeutic agent based on CTAs also comprises an immunotherapeutic agent specific for a CTA of the MAGE family. Preferably the CTA gene of the MAGE family is MAGE A2 and/or MAGE A3, more preferably is MAGE A2, and even more preferably MAGE A2 and MAGE A3.
As shown in Examples 1, 3 and 4, an increase in the expression level of a CTA gene of the non-X-CTA family is also detected, thus, in a particular embodiment of the second aspect of the invention according to any of the previous three paragraphs, the immunotherapeutic agent based on CTAs also comprises an immunotherapeutic agent specific for a non-X-CTA, preferably it comprises an immunotherapeutic agent specific for CTCFL.
In a preferred embodiment of the second aspect of the present invention, the CaSR activator agent is Cinacalcet; the CaSR-expressing cancer is selected from neuroblastomas, ganglioneuroblastomas, ganglioneuromas and combinations thereof; and more preferably the CaSR-expressing cancer is neuroblastoma; and the immunotherapeutic agent based on CTAs comprises immunotherapeutic agents specific for:
- a CTA of the SSX family, preferably SSX4 and/or SSX4B, and
- a CTA of the GAGE family, preferably selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 121, GAGE 12J, GAGE 13 and combinations thereof; more preferably GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, and optionally:
- a CTA of the MAGE family, preferably MAGE A2 and/or MAGE A3, and/or
- a non-X-family CTA, preferably CTCFL.
In an even more preferred embodiment of the second aspect of the invention, according to the previous paragraph, the immunotherapeutic agent based on CTAs comprises immunotherapeutic agents specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3 and CTCFL.

In a third aspect, the present invention refers to a kit comprising a CaSR activator agent and an immunotherapeutic agent based on CTAs as combination for the simultaneous, separate or sucessive administration. The kit of the third aspect of the invention is used for the treatment of a CaSR-expressing cancer. The CaSR activator agent and CTA-based immunotherapeutic agent may be formulated as compositions comprising a pharmaceutically acceptable carrier. Thus, in a particular embodiment, the kit comprises (1) a composition comprising a CaSR activator agent and (2) a composition comprising an immunotherapeutic agent based on CTAs, as a combination for their simultaneous, separate or sucessive administration.
The kit according to the third aspect of the invention is used for the treatment of a CaSR-expressing cancer. In a particular embodiment, the CaSR-expressing cancer is selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof. In a preferred embodiment, the CaSR-expressing cancer is selected from neuroblastomas, ganglioneuroblastomas, ganglioneuromas and combinations thereof; and more preferably is neuroblastoma.

In a particular embodiment of the kit of the third aspect of the invention, the CaSR activator agent is a CaSR allosteric activator. In a preferred embodiment, the CaSR activator agent is selected from Cinacalcet, NPS-R568, calindol, calcimimetic B and AC-265347 and combinations thereof, and in a more preferred embodiment the CaSR activator agent is Cinacalcet.
In a particular embodiment of the kit according to any one of the paragraphs above, the immunotherapeutic agent based on CTAs comprises an immunotherapeutic agent specific for a CTA of the SSX family, and/or an immunotherapeutic agent specific for a CTA of the GAGE family.
In a preferred embodiment of the kit according to the paragraph above, the immunotherapeutic agent based on CTAs comprises immunotherapeutic agents specific for:
- a CTA of the SSX family selected from SSX 4 and/or SSX 4B, and/or
- a CTA of the GAGE family selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof. In particular, 1, 2, 3, 4, 5, 6, 7, 8, 9, 10, 11, 12, 13, 14, 15, 16, 17, 18, 19, 20, 21 or 22 of the previously mentioned GAGEs. More preferably specific for GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. In a yet more preferred embodiment, the immunotherapeutic agent based on CTAs comprises immunotherapeutic agents specific for SSX 4 and/or SSX 4B; and, for GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. More preferably specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H and GAGE 12J.
In a particular embodiment of the kit according to any of the two previous paragraphs, the immunotherapeutic agent based on CTAs also comprises an immunotherapeutic agent specific for a CTA of the MAGE family. Preferably the CTA of the MAGE family is MAGE A2 and/or MAGE A3, more preferably it is MAGE A2, and even more preferably MAGE A2 and MAGE A3.
In another particular embodiment of the kit according to any of the previous three paragraphs, the immunotherapeutic agent based on CTAs also comprises an immunotherapeutic agent specific for a non-X-CTA gene, preferably it comprises an immunotherapeutic agent specific for CTCFL.
A preferred embodiment of the third aspect of the present invention refers to a kit comprising Cinacalcet and an immunotherapeutic agent based on CTAs comprising immunotherapeutic agents specific for:
- a CTA of the SSX family, preferably SSX4 and/or SSX4B, and
- a CTA of the GAGE family, preferably selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof; more preferably GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, and optionally:
- a CTA of the MAGE family, preferably MAGE A2 and/or MAGE A3, and/or
- a non-X-family CTA, preferably CTCFL.
In an even more preferred embodiment of the third aspect of the invention, according to the previous paragraph, the immunotherapeutic agent based on CTAs comprises immunotherapeutic agents specific for SSX 4, SSX 4B, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, MAGE A2, MAGE A3 and CTCFL. And more preferably specific for SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3 and CTCFL.

Furthermore, the inventors have surprisingly found that the increase of the expression level of a CTA gene after a CaSR activator agent is administered to a subject with a CaSR-expressing cancer, serves as biomarker of the effectiveness of the treatment with said CaSR activator agent. The best indication of a cancer response to a particular treatment is reduction of tumor volume or tumor growth. This is how response to chemotherapy is usually evaluated, given that tumor volume reduction and/or stability indicates massive tumor cell death and/or cell cycle exit and differentiation. However, dramatic tumor shrinkage does not occur upon cancer treatment with many so-called molecularly or biologically targeted therapies, thus making necessary to use surrogate markers to evaluate tumor response. As shown in the Examples, upregulation of the abovementioned CTAs is observed in neuroblastoma models exposed to Cinacalcet both *in vitro* and *in vivo* and this does not happen in normal cells. Thus, upregulation of the expression of these genes upon Cinacalcet treatment supports the use of CTAs as markers of cancer response to treatment with a CaSR activator agent. Analysis of these markers can be perfomed by direct analysis of the tumor (biopsy) and also in biological fluids, including peripheral blood, since CTAs circulate in blood both as proteins and mRNAs. Thus, the present invention also refers to a method for assesing the effectiveness of a treatment of a CaSR-expressing cancer with a CaSR activator agent by quantifying the level of expression of CTAs and to the use of CTAs as surrogate markers. Thus, in a fourth aspect, the present invention refers to an *in vitro* method for assessing the effectiveness of a therapy administered to a subject with a CaSR-expressing cancer, comprising the following steps:
a) quantifying the expression level of a CTA gene in a biological sample from said subject before and after the therapy, and
b) comparing the expression level of the CTA gene in the biological samples,
wherein the therapy administered comprises a CaSR activator agent and wherein an increase of the expression level of the CTA gene after the therapy with respect to the expression level of said CTA gene before the therapy is indicative that the therapy is effective.
In the context of the present invention the term "subject" refers to any member of the class Mammalia, including, without limitation, humans and nonhuman primates such as chimpazees and other apes and monkey species; farm animal such as catlle, sheep, pigs, goats and horses; domestic mammals such as dogs and cats; laboratory animals incluiding rodents such as mice, rats and guinea pigs, and the like. The term does no denote a particular age or sex. Thus, adult and newborn subjects, as well as fetuses, whether male or female, are intended to be included within the scope of this term. The subject is preferably a human.

The expression levels of the CTA gene in the biological samples can be quantified based on the RNA resulting from the transcription of said gene (mRNA) or, alternatively, based on the complementary DNA (cDNA) of said gene. Therefore, in a particular embodiment quantifying the expression levels of the CTA gene comprises quantifying the mRNA of the CTA gene, a fragment of said mRNA, cDNA of the CTA gene, a fragment of said cDNA, or mixtures thereof. Additionally, the method of the invention can include performing an extraction step for the purpose of obtaining the total RNA, which can be done by means of conventional techniques.
Virtually any conventional method can be used within the framework of the invention for detecting and quantifying the levels of mRNA encoded by the CTA gene or of its corresponding cDNA. By way of non-limiting illustration, the levels of mRNA can be quantified by means of the use of conventional methods, for example, methods comprising mRNA amplification and quantifying the product of said mRNA amplification, such as electrophoresis and staining, or alternatively by means of Northern blot and the use of specific probes of the mRNA of the gene of interest (CTA) or of its corresponding cDNA, mapping with S1 nuclease, RT-PCR, hybridization, microarrays, etc. Similarly, the levels of the cDNA corresponding to said mRNA encoded by the CTA gene can also be quantified by means of the use of conventional techniques. In this case, the method of the invention includes a step of synthesis of the corresponding cDNA by means of reverse transcription of the corresponding mRNA followed by amplification and quantification of the product of said cDNA amplification. Conventional methods for quantifying the expression levels can be found, for example, in Sambrook et al., 2001. "Molecular cloning: a Laboratory Manual", 3rd ed., Cold Spring Harbor Laboratory Press, N.Y., Vol. 1-3.
The expression levels of the CTA gene in the biological samples can be quantified based on the protein encoded by said gene, i.e. the CTA protein, or any amino acid sequence that has at least 90% identity with the protein encoded by the CTA gene, still more preferably with at least 95%, 96%, 97%, 98% or 99% identity with respect to said protein. The degree of identity between two amino acid sequences can be determined by conventional methods, for example, by means of standard sequence alignment algorithms known in the state of the art, such as, for example, BLAST (Altschul S. et al. Basic local alignment search tool. J Mol Biol. 1990. 215(3):403-410). The expression level of the CTA protein can be quantified by means of any conventional method which allows detecting and quantifying said protein in a sample from a subject. There is a wide variety of known assays which can be used in the present invention, these techniques include Western blot, ELISA (enzyme-linked immunosorbent assay), RIA (radioimmunoassay), competitive EIA (competitive enzymatic immunoassay), DAS-ELISA (double antibody sandwich ELISA), immunocytochemical and immunohistochemical techniques, techniques based on the use of protein biochips or microarrays including specific antibodies or assays based on colloidal precipitation. Other ways for detecting and quantifying said CTA protein include affinity chromatography techniques, ligand binding assays, etc. In a particular embodiment, the level of protein encoded by the CTA gene are quantified by means of Western blot, immunohistochemistry or ELISA.
In a particular embodiment of the method according to the fourth aspect of the invention, in step a) the expression level is quantified by any one method selected from:
a) detecting mRNA of a CTA gene;
b) detecting the protein encoded by a CTA gene; and
c) detecting any amino acid sequence that has at least 90% identity with a protein encoded by a CTA gene.
In yet a more preferred embodiment, in step a) the expression level is quantified by detecting mRNA of a CTA gene, preferably by RTqPCR or by microarrays.
Putting the method of the invention into practice comprises obtaining a biological sample from the subject to be studied. CTAs are expressed in tumor tissue samples and they can also be detected in biological fluids of subjects diagnosed with cancer. Therefore, in a particular embodiment, the biological sample is a tumor tissue sample or a biological fluid, and in yet another more particular embodiment is a biological fluid, so the method is less invasive than the ones requiring a tumor tissue sample taken by means of biopsy. In a preferred embodiment, the biological sample is a biological fluid selected from blood, serum, plasma, peritoneal fluid, pericardic fluid, pleural fluid, cerebrospinal fluid and intra-articular fluid, preferably the biological fluid is selected from blood, serum and plasma, and more preferably the biological sample is blood.
In a particular embodiment of the method according to the fourth aspect of the present invention as defined in the paragraphs above, the CaSR activator agent is an allosteric activator of the CaSR, in a preferred embodiment the CaSR activator agent is selected from Cinacalcet, NPS-R568, calindol, calcimimetic B, AC-265347 and combinations thereof, and more preferably the CaSR activator agent is Cinacalcet. In another particular embodiment of the method according to the fourth aspect of the invention as defined in any of the paragraphs above, the CaSR-expressing cancer is selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof. In a preferred embodiment, the CaSR-expressing cancer is selected from neuroblastomas, ganglioneuroblastomas, ganglioneuromas and combinations thereof; and more preferably is neuroblastoma.
One of the limitations of immunotherapies based on CTAs is the notable degree of intra- and inter-tumoral heterogeneity of CTAs expression. This makes reasonable to simultaneously analyze several markers of this family of genes/antigens. In the data described in the Examples, the most consistently upregulated markers were those of the GAGE and SSX families. Thus, in a particular embodiment of the method of the fourth aspect of the invention according to any one of the paragraphs above, step a) comprises quantifying the expression level of a CTA gene of the SSX family, and/or a CTA gene of the GAGE family. Preferably, the CTA gene of the SSX family is SSX 4 and/or SSX 4B, more preferably are SSX 4 and SSX 4B, and the CTA gene of the GAGE family is selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof. In particular, 1, 2, 3, 4, 5, 6, 7, 8, 9, 10, 11, 12, 13, 14, 15, 16, 17, 18, 19, 20, 21 or 22 of the previously mentioned GAGEs. More preferably the CTAs of the GAGE family are GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. More preferably, in the method of the fourth aspect, step a) comprises quantiying the expression level of the CTAs of the SSX family and GAGE family as described above in this paragraph, and more preferably, comprises quantiying the expression level of SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H and GAGE 12J. Quantification of a combination of CTAs would produce the most robust results taking into account the heterogenous pattern of gene expression that is present in tumors before therapy, and thus probably also upon therapy with CaSR activating agents. Thus, in another preferred embodiment of the fourth aspect of the invention according to the previous paragraph, step a) also comprises quantifying the expression level of a CTA gene of the MAGE family, preferably MAGE A2 and/or MAGE A3, more preferably MAGE A2 and even more preferably MAGE A2 and MAGE A3.
In another preferred embodiment of the fourth aspect of the invention according to any of the two previous paragraphs, step a) also comprises quantifying the expression level of a non-X-CTA gene, preferably of CTCFL.
In a preferred embodiment, step a) comprises quantifying the expression level of the following CTA genes: SSX 4, SSX 4B, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, MAGE A2, MAGE A3 and CTCFL. And more preferably, SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3 and CTCFL.

In a fifth aspect, the present invention refers to a kit for carrying out the method according to any of the embodiments of the fouth aspect of the invention, comprising appropriate means to measure the expression level of the CTA genes, and a suitable packaging. The term "packaging" refers to the containment and packing prior to sale with the primary purpose of facilitating the purchase and use of a product.
In a particular embodiment of the fifth aspect, the kit comprises appropriate means to measure the expression level of a CTA gene of the SSX family and of a CTA gene of the GAGE family. Preferably, the kit comprises appropriate means to measure the expression level of a CTA gene of the SSX family selected from SSX 4 and/or SSX 4B, and a CTA gene of the GAGE family selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof. In particular, 1, 2, 3, 4, 5, 6, 7, 8, 9, 10, 11, 12, 13, 14, 15, 16, 17, 18, 19, 20, 21 or 22 of the previously mentioned GAGEs. More preferably GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. In a yet more preferred embodiment, the kit of the fifth aspect comprises appropriate means to measure the expression level of the CTAs: SSX 4, SSX 4B, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, and more preferably SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H and GAGE 12J. In another preferred embodiment of the fifth aspect of the invention according to the previous paragraph, the kit comprises appropiate means to measure the expression level of a CTA gene of the MAGE family. Preferably the CTA gene of the MAGE family is MAGE A2 and/or MAGE A3, more preferably is MAGE A2, and even more preferably MAGE A2 and MAGE A3.
In another preferred embodiment of the fifth aspect of the invention according to any of the two previous paragraphs, the kit comprises appropiate means to measure the expression level of a non-X-CTA gene, preferably of CTCFL.
In a more preferred embodiment of the fifth aspect of the invention, the kit comprises appropriate means to measure the expression of the CTAs: SSX 4, SSX 4B, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, MAGE A2, MAGE A3 and CTCFL. And more preferably, SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3 and CTCFL.
Appropriate means to measure the expression can be commercially available or produced by the skilled in the art, such as CTA-specific primers, probes, antibodies. Appropriate means are, amongs others, the ones described in the Examples of the present application, particularly the ones described in Table 1, specially the primers of sequence SEQ ID NO 1- SEQ ID NO 20, which are specific for the CTAs depicted in said Table 1.

As shown in the Examples, both *in vitro* and *in vivo,* and using two independent techniques, CTAs were found to be upregulated upon Cinacalcet treatment in neuroblastoma models. Other molecules were upregulated as well (for instance, *NTRK3, PRKCA* and *RYR2,* see Examples 1 and 3). However, these genes are also expressed in normal tissues while CTAs are almost exclusively overexpressed in tumor cells. This makes CTAs the most preferable markers of tumor response to a treatment with a CaSR activator among all the upregulated genes in these cancer models. Thus, in a sixth aspect, the present invention refers to the use of a CTA, or to the use of the level of expression of a CTA, as surrogate marker of tumor response of a CaSR-expressing cancer to treatment with a CaSR activator agent. Increasing levels of these surrogate markers will indicate that the CaSR activator agent is effectively carrying our its function on CaSR-expressing cancer cells despite absence of dramatic or rapid changes in tumor volume.
In a particular embodiment of the use according to the sixth aspect of the invention, the CaSR activator agent is an allosteric activator of CaSR, in a preferred embodiment the CaSR activator agent is selected from Cinacalcet, NPS-R568, calindol, calcimimetic B, AC-265347 and combinations thereof, and more preferably the CaSR activator agent is Cinacalcet.
In another particular embodiment of the use according to the sixth aspect of the invention as defined in any of the paragraphs above, the CaSR-expressing cancer is selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof. In a preferred embodiment, the CaSR-expressing cancer is selected from neuroblastomas, ganglioneuroblastomas, ganglioneuromas and combinations thereof; and more preferably is neuroblastoma.
A preferred embodiment of the sixth aspect of the present invention, the CTA used is a CTA of the SSX family, preferably SSX4 and/or SSX4B; and/or a CTA of the GAGE family, preferably selected from GAGE 1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof. In particular, 1, 2, 3, 4, 5, 6, 7, 8, 9, 10, 11, 12, 13, 14, 15, 16, 17, 18, 19, 20, 21 or 22 of the previously mentioned GAGEs. More preferably the CTAs of the GAGE family are GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof. In a yet more preferred embodiment, the CTAs used are: SSX 4, SSX 4B, GAGE 1, at least one of GAGE 2A, GAGE 2B, GAGE 2C, GAGE 2D, GAGE 2E and combinations thereof, GAGE 10, and at least one of GAGE 12B, GAGE 12C, GAGE 12D, GAGE 12E, GAGE 12G, GAGE 12H, GAGE 12J and combinations thereof, MAGE A2, MAGE A3 and CTCFL. And more preferably, SSX 4, SSX 4B, GAGE 1, GAGE 2A, GAGE 2B, GAGE 2C, GAGE 2D, GAGE 2E, GAGE 10, GAGE 12B, GAGE 12C, GAGE 12D, GAGE 12E, GAGE 12G, GAGE 12H, GAGE 12J, MAGE A2, MAGE A3 and CTCFL.
In a yet more preferred embodiment according to any of the embodiments described in the paragraph above, a CTA of the MAGE family, preferably MAGE A2 and/or MAGE A3, more preferably MAGE A2 and even more preferably MAGE A2 and MAGE A3; and/or a CTA of the non-X-family, preferably CTCFL, are also used.

The examples below serve to further illustrate the invention, and to provide those of ordinary skill in the art with a complete disclosure and description of how the uses and methods herein are carried out, and are not intended to limit the scope of the invention.

### EXAMPLES

### Example 1. Cinacalcet induces cytodifferentiation and upregulation of CTAs in neuroblastoma in vitro models

Three neuroblastoma cell lines LA-N-1, SK-N-LP and SH-SY5Y were used for this purpose. We have already reported that LA-N-1 cells exhibit endogeneous CaSR expression, although at low levels, while the *CaSR* gene is silenced by epigenetic mechanisms in SK-N-LP cells (Casalà C, et al. The calcium-sensing receptor gene is silenced by genetic and epigenetic mechanisms in unfavorable neuroblastomas and its reactivation induces ERK1/2-dependent apoptosis. Carcinogenesis 2013; 34:268-76). No expression of the *CaSR* is detectable in SH-SY5Y either. Thus, SK-N-LP and SH-SY5Y were stably transfected with pCMV-CaSR-GFP or pCMV-GFP.

All cell lines were grown in the presence of 1 µM Cinacalcet (Selleck Chemicals, S1260) or DMSO for 21 days in P100 plates. Morphology was assessed daily. Total RNA was isolated at days 1, 3, 5, 7, 10, 14 and 21. Several markers were analyzed by RT-qPCR using SYBRgreen and specific primers or Taqman technology and gene-specific Assays-on-Demand (Applied Biosystems) (Table 1).

**Table 1.-Assays on Demand and primers used for expression analyses by RT-qPCR.**

| **Gene** | **Assay on Demand (ABI)** | **Primer forward (5'-3')** | **Primer reverse (5'-3')** | **mRNAs** |
|---|---|---|---|---|
| ***NTRK1*** | Hs01021011_m1 | | | |
| ***NTRK3*** | Hs00176797_m1 | | | |
| ***NFL*** | Hs00196245_m1 | | | |
| ***TUBB3*** | Hs00801390_s1 | | | |
| ***S100-β*** | Hs00902901_m1 | | | |
| ***CTCFL*** | | gcacggaaaagcgacctacg (SEQ ID NO 1) | tgagggcatagcgttcatgg (SEQ ID NO 2) | Homo sapiens CCCTC-binding factor (zinc finger protein)-like (CTCFL), transcript variant 1-9, 11-12, 15-17 |
| ***SSX4*/*4B*** | | caccctcccacctttcatgc (SEQ ID NO 3) | ggacgttcaacctgattcctgtg (SEQ ID NO 4) | Homo sapiens synovial sarcoma, X breakpoint 4 (SSX4), transcript variant 1 and 2, breakpoint 4B (SSX4B), transcript variant 1 and 2 |
| ***GAGE-P1*** | | gcagctgctcaggagggaga (SEQ ID NO 5) | tgtgggtgaccctgttcctg (SEQ ID NO 6) | See below |
| ***GAGE-P2*** | | ggccgaagcctgaagctcat (SEQ ID NO 7) | atttggcgggtccatctcct (SEQ ID NO 8) | See below |
| ***GAGE-P3*** | | gagggagcatctgcaggtcaa (SEQ ID NO 9) | cctgcccatcaggaccatct (SEQ ID NO 10) | See below |
| ***MAGE A2*** | | aaccaggcagtgaggccttg (SEQ ID NO 11) | agcctgtccccctcagaacc (SEQ ID NO 12) | Homo sapiens melanoma antigen family A2 (MAGEA2), transcript variant 3-4 |
| ***MAGE A3*** | | aaggtggccgagttggttca (SEQ ID NO 13) | actgccaatttccgacgaca (SEQ ID NO 14) | Homo sapiens melanoma antigen family A3 (MAGEA3), variant X1-4 |
| ***ADCY8*** | | cgccaagctttcctggagac (SEQ ID NO 15) | acgagccgctcctgtctttg (SEQ ID NO 16) | Homo sapiens adenylate cyclase 8 (brain) (ADCY8), transcript variant X1-2 |
| ***PRKCA*** | | ctgagaagagggggcggatt (SEQ ID NO 17) | ggatctgaaagcccgtttgg (SEQ ID NO 18) | Homo sapiens protein kinase C, alpha (PRKCA), transcript variants X 1-4 |
| ***RYR2*** | | gctcaaggtggtggtcatcg (SEQ ID NO 19) | ttgaagaccgggaggtggac (SEQ ID NO 20) | Homo sapiens ryanodine receptor 2 (cardiac) (RYR2), transcript variant X |

| | | | | |
|---|---|---|---|---|
| (*) All mRNA variants detected unless otherwise specified. GAGE P1: Homo sapiens G antigen (GAGE): 12B, 2B, 12D, 12E, 12C, 12G, 12C, 2C, 1 (transcript variant X1), 12J, 1 (transcript variant 3, non-coding RNA), 1 (transcript variant 2), 2A, 2E, 12H, 2D, 10. GAGE P2: Homo sapiens G antigen (GAGE): 12B, 2B, 2A, 12D, 12E, 12C, 12H, 12G, 2C, 1 (transcript variant X1), 12J, 1 (transcript variant 3, non-coding RNA), 1 (transcript variant 2). GAGE P3: Homo sapiens G antigen (GAGE): 12B, 2B, 2A, 12D, 12E, 12C, 12H, 12G, 1 (transcript variant X1), 12J, 1 (transcript variant 3, non-coding RNA), 1 (transcript variant 2), 2E, 2D, 10. | | | | |

Morphological features of neuronal differentiation such as extensive neurite outgrowth were already apparent at day 3. These features reached maximal expression between days 7 and 14 (Figure 1). Analysis of mRNA expression by RT-qPCR showed upregulation of genes associated with neuroblastoma cytodifferentiation and several genes encoding CTAs (Table 2). Neuroblastoma cytodifferentiation is an indication of this tumor becoming more benign, less proliferative. Thus, upregulation of differentiation-associated genes shows that Cinacalcet exerts anti-tumoral effects in the context of neuroblastoma. Moreover, upregulation of these differentiation-associated genes and CTAs both *in vitro* and *in vivo* (see Examples 2-4) validates the proposed *in vitro* model (doses used, time of exposure, etc) as a good model to evaluate neuroblastoma response to treatment with Cinacalcet.

**Table 2.- Upregulated genes in neuroblastoma in vitro models following treatment with Cinacalcet.**

| | | **LA-N-1** | **SK-N-LP CaSR/GFP** | **SH-SY5Y CaSR/GFP** |
|---|---|---|---|---|
| | **Gene** | **Fold increase** | | |
| **Differentiation** | ***NTRK1*** | 3.2 | 3.6 | 2.9 |
| | ***NTRK3*** | 2.4 | 2.6 | 4.7 |
| | ***NFL*** | ns | 7.2 | 2.3 |
| | ***TUBB3*** | ns | 2.8 | 1.7 |
| | ***S100-β*** | 6.9 | 72.3 | 2.2 |
| **CTAs** | ***CTCFL*** | 1.2 | no expression | no expression |
| | ***SSX4*/*4B*** | 3.8 | 69 | 3.45 |
| | ***GAGE-P1*** | 2.7 | 4.1 | 1.4 |
| | ***GAGE-P2*** | 7.6 | 5.7 | 7.9 |
| | ***GAGE-P3*** | 5.1 | 6.3 | 2.5 |
| | ***MAGEA2*** | 2.9 | 36.6 | 2.4 |
| | ***MAGEA3*** | 1.4 | 155.4 | 14.6 |

| | | | | |
|---|---|---|---|---|
| ns: not significant. | | | | |

### Example 2. Cinacalcet inhibits neuroblastoma tumor growth in vivo

An *in vivo* model of neuroblastoma was generated in four to six-week-old female athymic Nude-Foxn1 *nu*/*nu* mice (Charles Rivers), wherein LA-N-1 cells (10⁷) were subcutaneously inoculated. Tumors were allowed to grow until dimensions reached 7x7 mm. Then mice were randomized to receive either vehicle (described below) or Cinacalcet (10 mg/kg/day) by oral gavage 6 days per week until tumor volume reached 2 cm³. Cinacalcet was prepared with Mimpara® tablets (Amgen). Following homogenization of the Mimpara® tablets in a mortar, the resulting powder was reconstituted with 0.5% Tween 20 (20%, v/v) and carboxymethylcellulose (0.25%, w/v) to a final concentration of 20 mg/mL. Each dose contained 5 µL of this mixture per gram of mouse weight. Dimensions of tumors were measured thrice a week using a digital caliper. Tumor volume was calculated as L x W²/2 in which "L" indicates length in mm and "W" indicates width in mm. At the end of the experiment (each mouse was sacrificed when the corresponding xenograft reached the final predetermined volume 2 cm³), tumors were excised and half of each specimen was frozen in liquid nitrogen for molecular analyses and the other was fixed in 10% formalin. For event-free survival (EFS) analysis, an event was defined as tumor size that exceeded 2 cm³. The log-rank statistic was used to compare the EFS probabilities between groups. *P*<0.05 was considered significant.

As shown in Figure 2A, significant tumor growth inhibition was observed in mice treated with Cinacalcet as compared to controls (*P*=0.034). Mild, non-symptomatic, hypocalcemia was documented in mice treated with Cinacalcet (Figure 2B). This result is the expected consequence of Cinacalcet action on parathyroid glands and kidneys, and thus a good indication of the drug being correctly administered and absorbed. No signs of toxicity were observed (not shown): mice were eating and growing normally, and mild hypocalcemia was well tolerated.

Overall, this murine model showed that prolonged exposure to Cinacalcet reduces neuroblastoma tumor growth *in vivo,* being this anti-tumoral effect obtained at well-tolerated doses.

### Example 3. Upregulation of CTAs in neuroblastoma upon Cinacalcet in vivo treatment: Microarrays analyses

Total RNA was isolated from 8 xenografts exposed to Cinacalcet (C) and 8 from the control group (V). These were the last 8 tumors excised in each group, so that analyzed xenografts were those exposed to the longest period of treatment (V5-V12 and C4-C11). Two different mRNA expression analyses were conducted: Genome-wide expression analysis by microarrays (Example 3) and by RT-qPCR (Example 4). Genome-wide expression analyses were performed using Affymetrix Human and Mouse Gene Array 2.1 ST (Affymetrix, reference number 902136 for human gene array and 902120 for mouse gene array) as per manufacturer's protocol. Procedures to analyze results included: Quality control of the arrays (following this step, xenograft C6 was removed from further analyses); filtering and normalization; selection of differentially expressed genes; single comparison (treated *versus* control samples); list of differentially expressed genes ranked by significance; Volcano plots showing the most differentially expressed genes; heatmaps of the clustered expression profiles; and analysis of biological significance through enrichment analysis against the Gene Ontologies, KEGG (Kyoto Encyclopedia of Genes and Genomes) and Ingenuity pathways.

Initial genome-wide expression analysis identified a group of 13 probes corresponding to potentially upregulated genes (log fold change >1) in Cinacalcet-treated tumors (C4, C5, C7-C11) as compared to controls (V5-V12). Interestingly, 11 out of these 13 probes hybridized with CTAs genes (bold, Table 3). However, these differences of gene expression were not statistically significant, adjusted *P*-value > 0.05 (Table 3). Nonetheless, in this first analysis, corresponding to heatmap on Figure 3, it was apparent that a group of genes were specifically modulated in the three tumors exposed to Cinacalcet for the longest period of time. This was confirmed by a second genome-wide expression analysis that examined differentially expressed genes in these three specimens (C9-C11) compared to the 8 vehicle-treated tumors (V5-V12) (Figure 4).

**Table 3.- Upregulated genes in Cinacalcet-treated xenografts versus vehicle-treated xenografts (log fold change > 1)**

| **Probe** | **SymbolsA** | **Entrez** | **Log fold change** | **Adjusted P. alue** |
|---|---|---|---|---|
| 17103626 | **GAGE12G//GAGE12F//GAGE12I//GAGE7//GAGE6//GAGE4//GAGE5//GAGE12C//GAGE12D//GA GE12E//GAGE12H//GAGE12J//GAGE2E//GAGE8//GAGE12B//GAGE2D//GAGE2B//GAGE2C//GA GE13//GAGE2A//GAGE1** | 645073 | 1.48896708 | 0.39648602 |
| 17104821 | CXorf49//CXorf49B | 100130361 | 1.42318655 | 0.61235652 |
| 17103653 | **GAGE12C//GAGE12D//GAGE12E//GAGE12G//GAGE12H//GAGE12J//GAGE2A//GAGE2E//GAGE 8//GAGE10//GAGE12B//GAGE2C//GAGE13//GAGE6//GAGE12F//GAGE12I//GAGE7//GAGE4//GA GE2D//GAGE5//GAGE2B** | 729422 | 1.3539287 | 0.52209349 |
| 17103647 | **GAGE2A//GAGE2C//GAGE2B//GAGE2D//GAGE2E//GAGE8//GAGE12F//GAGE12G//GAGE12I//G AGE7//GAGE5//GAGE6//GAGE4//GAGE12J//GAGE12C//GAGE12D//GAGE12E//GAGE12H//GAG E12B//GAGE13** | 729447 | 1.34133487 | 0.4625512 |
| 17103679 | **GAGE2A//GAGE2C//GAGE12G//GAGE12F//GAGE12I//GAGE7//GAGE5//GAGE2B//GAGE2D//GA GE12C//GAGE12D//GAGE12E//GAGE12H//GAGE2E//GAGE8//GAGE12J//GAGE6//GAGE4//GAG E12B//GAGE13//GAGE1** | 729447 | 1.31803532 | 0.47520907 |
| 17103620 | **GAGE13//GAGE12D//GAGE12E//GAGE12B//GAGE12C//GAGE12H//GAGE12J//GAGE2E//GAGE 8//GAGE2D//GAGE6//GAGE12F//GAGE12G//GAGE12I//GAGE7//GAGE4//GAGE1//GAGE5//GAG E2A//GAGE2C//GAGE2B** | 645051 | 1.31383129 | 0.52209349 |
| 17103655 | **GAGE12C//GAGE12D//GAGE12E//GAGE12H//GAGE12G//GAGE12B//GAGE12F//GAGE12I//GAG E7//GAGE5//GAGE12J//GAGE6//GAGE4//GAGE13//GAGE2E//GAGE8//GAGE2D//GAGE2A//GAG E2C//GAGE2B//GAGE1** | 729422 | 1.31309793 | 0.52209349 |
| 17103690 | **GAGE12H//GAGE12C//GAGE12D//GAGE12E//GAGE12G//GAGE12B//GAGE12F//GAGE12I//GAG E7//GAGE6//GAGE4//GAGE5//GAGE12J//GAGE2D/IGAGE13//GAGE2E//GAGE8//GAGE2A//GAG E2C//GAGE2B//GAGE1** | 729442 | 1.26052266 | 0.52209349 |
| 17103673 | **GAGE12G//GAGE12C//GAGE12D//GAGE12E//GAGE12H//GAGE12F//GAGE12I//GAGE7//GAGE6 //GAGE4//GAGE5//GAGE12J//GAGE2A//GAGE2D//GAGE2C//GAGE2B//GAGE12B//GAGE2E//G AGE8//GAGE13//GAGE1** | 645073 | 1.25675401 | 0.52209349 |
| 17103614 | **GAGE12J//GAGE2D//GAGE2E//GAGE8//GAGE2A//GAGE2C//GAGE6//GAGE2B//GAGE1//GAGE 13//GAGE12G//GAGE4//GAGE5//GAGE12F//GAGE12I//GAGE7//GAGE12C//GAGE12D//GAGE12 E//GAGE12H//GAGE12B** | 729396 | 1.19149006 | 0.52209349 |
| 16920617 | **CTCFL** | 140690 | 1.13688699 | 0.61235652 |
| 16902703 | **POTEF** | 728378 | 1.12397293 | 0.35366385 |
| 16837226 | SNORA38B | 100124536 | 1.10662073 | 0.35366385 |

This second analysis identified a group of genes significantly modulated in Cinacalcet-treated specimens. This gene signature (or group of genes differentially expressed) encompassed 34 probes which hybridized with genes that were significantly upregulated in the Cinacalcet-treated group (log fold change >1, adjusted *P*-value < 0.05, Table 4). Among these 34 probes, 11 (32%) hybridized with CTAs (bold). These were CTAs encoded by genes located at the X chromosome (*GAGE* family and *SSX4*/*4B*)*.* The GAGE family was found to be upregulated in the first analysis as well (Table 3).

Other significantly upregulated genes in this second analysis included *PRKCA, RYR2* and *GABRA3.* The first two encode proteins that are important in the signaling pathways activated downstream of the CaSR, and GABA receptors are upregulated in neuroblastic tumors of good outcome.

Two other relevant genes (*CTCFL* and *NTRK3*) were also found to be upregulated in this second analysis, although adjusted *P*-value was right below the cut-off value (0.0514678 for both *CTCFL* and *NTRK3*). CTCFL (also called BORIS) is a non-X-CTA that has been shown to control the expression of other CTAs in some contexts, and NTRK3 is a receptor of the Trk family, that has been showed to be overexpressed in benign, good outcome, neuroblastic tumors.

Overall, this second analysis of microarrays data showed that neuroblastoma tumors become more benign, differentiated, when exposed to Cinacalcet for extended periods of time, and identified a surprising effect, totally unexpected, of the CaSR activating agent: a significantly upregulation of CTAs in neuroblastoma cells upon administration of said drug.

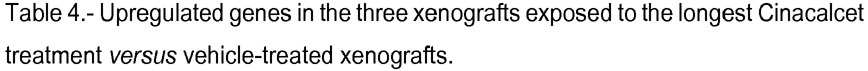

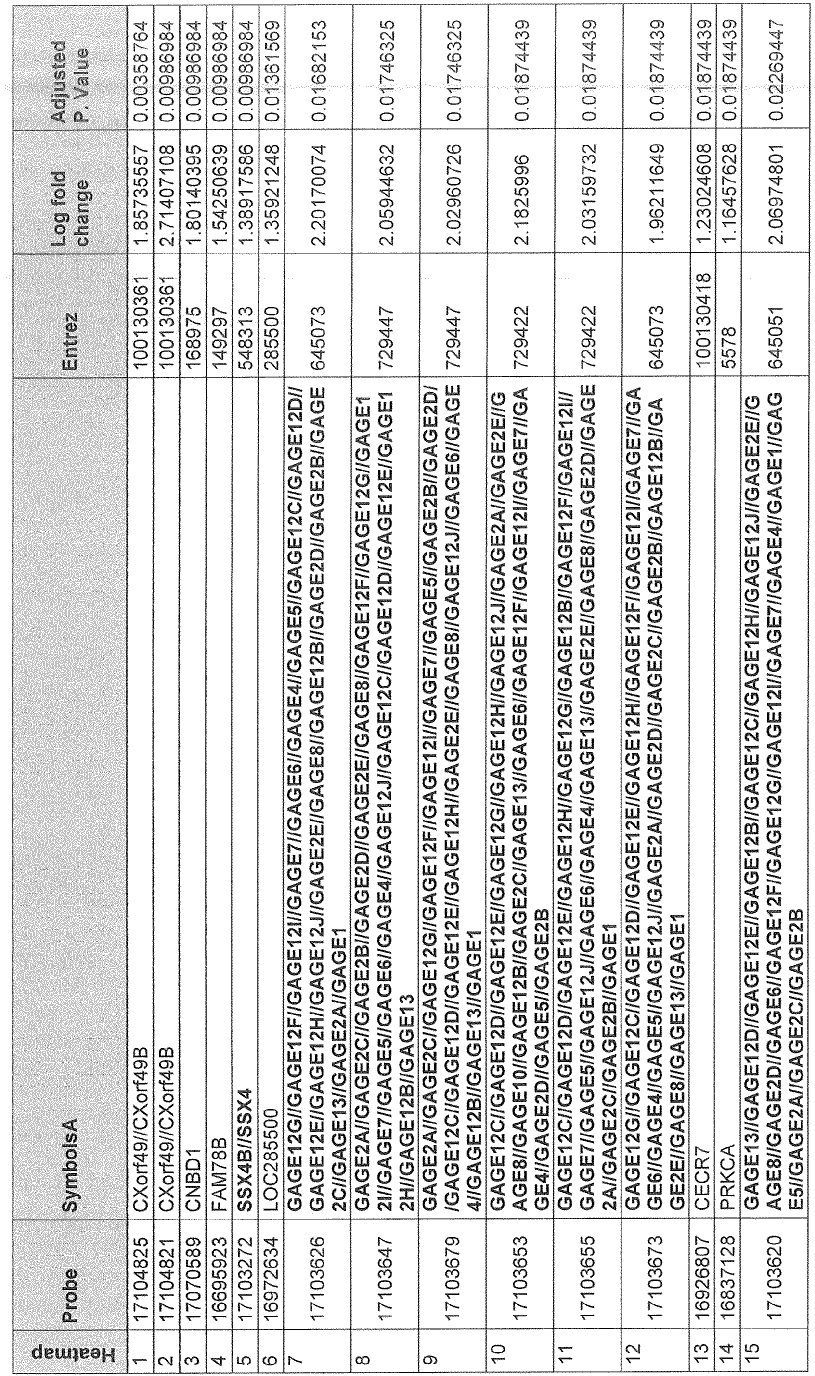

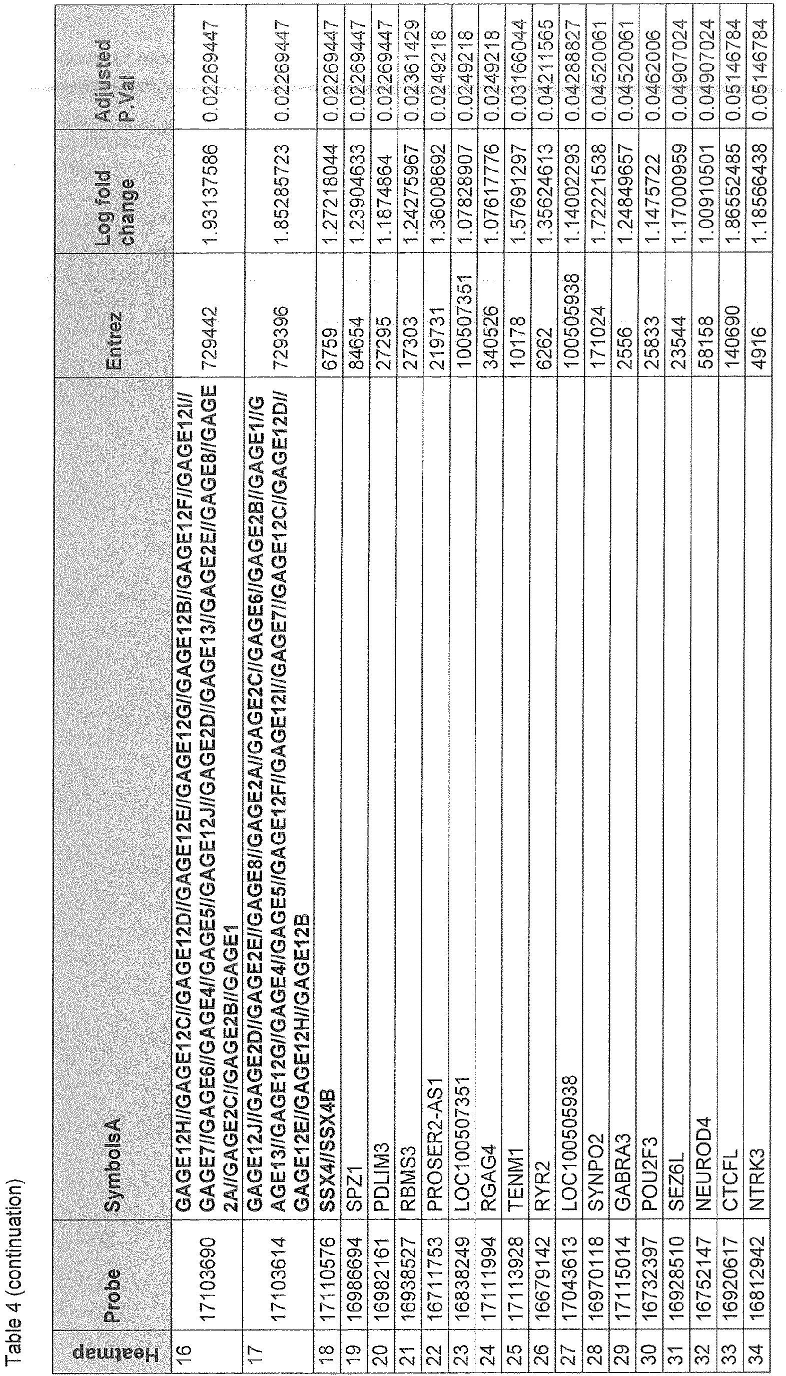

### Example 4. Upregulation of CTAs in neuroblastoma upon Cinacalcet in vivo treatment: RT-qPCR analyses

Validation of microarrays data was conducted by means of RT-qPCR. This technique was performed using SYBRgreen and specific primers or Taqman technology and gene-specific Assays-on-Demand (Applied Biosystems) (Table 1).

This technique was also used to analyze the expression of other CTAs (members of the *MAGE* family and *CTCFL*), genes associated with differentiation and calcium-signaling pathways in further detail. Upregulation of *CTCFL* (Figure 5A), *MAGE A2, MAGE A3* and *SSX4*/*4B* (Figure 5B), *NTRK3* (Figure 5C), *ADCY8, PRKCA* and *RYR2* (Figure 5D) was detected mainly in the two specimens exposed for the longest time to Cinacalcet (C10-C11).

Differential expression was also examined in the entire cohort of animals included in this experiment, wherein an increased up to 100-fold in the relative mRNA expression of *GAGE* mRNA in the specimen that received long-term treatment with Cinacalcet (C11) was observed (data not shown).

### Example 5. Cinacalcet does not induce upregulation of CTAs in normal surrounding tissues

In order to evaluate whether upregulation of CTAs was a tumor-specific effect or it was induced in normal surrounding tissues as well, a genome-wide expression analysis by means of microarrays was conducted using a chip to detect murine genes differentially expressed in the same RNAs analyzed in the human array. As show in Table 5, very few genes were upregulated (log fold change > 1), none of them significantly (adjusted *P*-value > 0.05), and CTAs were not amongst the overexpressed genes, no even when the three last specimens C9-C11 were analyzed. Thus, interestingly, Cinacalcet induces overexpression of CTAs in tumors, but not in surrounding non-neoplastic tissues, providing suitable targets for an immunotherapeutic agent.

**Table 5.- Mus musculus gene expression analysis by means of microarrays: Upregulated genes**

| **Probe** | **Symbols** | **Entrez** | **Log fold change** | **Adjusted P.Value** |
|---|---|---|---|---|
| 17386477 | Gm11084 | --- | 1.5672508 | 0.80678117 |
| 17546774 | Erdr1//Dock9 | 170942 | 1.3407767 | 0.80678117 |
| 17483098 | Gdpd3 | 68616 | 1.30938806 | 0.80678117 |
| 17458439 | Gpnmb | 93695 | 1.22129554 | 0.80678117 |
| 17262250 | Tgtp2//Tgtp1 | 100039796 | 1.10813814 | 0.80678117 |
| 17302054 | Snora31 | 100303751 | 1.10492833 | 0.80678117 |
| 17546762 | LOC101055644//Mid1//MID1 | 101055644 | 1.08780566 | 0.80678117 |
| 17395227 | Gm14399 | 100043761 | 1.0665113 | 0.80678117 |
| 17503825 | Mmp2 | 17390 | 1.04602291 | 0.80678117 |
| 17352957 | Dsc1 | 13505 | 1.03318783 | 0.80678117 |
| 17380529 | Gm14419//Gm14391//Gm6710 | 627901 | 1.03250936 | 0.80678117 |

## Claims

1. CaSR activator agent for use in increasing the susceptibility of a subject with a CaSR-expressing cancer to an immunotherapeutic agent based on CTAs.

2. CaSR activator agent for use according to claim 1, wherein the CaSR activator agent is a CaSR allosteric activator, preferably selected from Cinacalcet, NPS-R568, calindol, calcimimetic B, AC-265347 and combinations thereof; and more preferably the CaSR activator agent is Cinacalcet.

3. CaSR activator agent for use according to claim 1 or 2, wherein the cancer cell is a cell from a cancer selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof; preferably selected from neuroblastomas, ganglioneuroblastomas and ganglioneuromas; and more preferably is neuroblastoma.

4. CaSR activator agent for use according to any one of claims 1 to 3, for use in increasing the susceptibility to an immunotherapeutic agent based on a CTA of the SSX family and/or to an immunotherapeutic agent based on a CTA of the GAGE family.

5. CaSR activator agent for use according to claim 4, for use in increasing the susceptibility to an immunotherapeutic agent based on a CTA of the MAGE family and/or to an immunotherapeutic agent based on a non-X-family CTA.

6. A CaSR activator agent in combination with an immunotherapeutic agent based on CTAs for use in the treatment of a CaSR-expressing cancer.

7. A CaSR activator agent for use according to claim 6, wherein the CaSR activator agent is a CaSR allosteric activator, preferably is selected from Cinacalcet, NPS-R568, calindol, calcimimetic B, AC-265347 and combinations thereof; and more preferably the CaSR activator agent is Cinacalcet.

8. CaSR activator agent for use according to claim 6 or 7, wherein the cancer cell is a cell from a cancer selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof; preferably selected from neuroblastomas, ganglioneuroblastomas and ganglioneuromas; and more preferably is neuroblastoma.

9. A CaSR activator agent for use according to any one of claims 6 to 8, wherein the immunotherapeutic agent based on CTAs comprises an immunotherapeutic agent specific for:
- a CTA of the SSX family; preferably selected from SSX 4 and/or SSX 4B, and/or
- a CTA of the GAGE family; preferably selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof.

10. A CaSR activator agent for use according to claim 9, wherein the immunotherapeutic agent based on CTAs comprises an immunotherapeutic agent specific for a CTA of the MAGE family; preferably MAGE A2 and/or MAGE A3.

11. A CaSR activator agent for use according to claim 9 or 10, wherein the immunotherapeutic agent based on CTAs comprises an immunotherapeutic agent specific for a non-X-CTA; preferably CTCFL.

12. Kit comprising a CaSR activator agent and an immunotherapeutic agent based on CTAs as combination for simultaneous, separate or successive administration.

13. Kit according to claim 12, wherein the CaSR activator agent is a CaSR allosteric activator, preferably is selected from Cinacalcet, NPS-R568, calindol, calcimimetic B, AC-265347 and combinations thereof; and more preferably the CaSR activator agent is Cinacalcet.

14. Kit according to claim 12 or 13, wherein the immunotherapeutic agent based on CTAs comprises immunotherapeutic agents specific for:
- a CTA of the SSX family; preferably SSX 4 and/or SSX 4B, and/or
- a CTA of the GAGE family; preferably selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 121, GAGE 12J, GAGE 13 and combinations thereof.

15. Kit according to claim 14, wherein the immunotherapeutic agent based on CTAs comprises an immunotherapeutic agent specific for a CTA of the MAGE family; preferably MAGE A2 and/or MAGE A3.

16. Kit according to claim 14 or 15, wherein the immunotherapeutic agent based on CTAs comprises an immunotherapeutic agent specific for a non-X-CTA; preferably CTCFL.

17. An *in vitro* method for assessing the effectiveness of a therapy administered to a subject with a CaSR expressing cancer, comprising the following steps:
a) quantifying the expression level of a CTA gene in a biological sample from said subject before and after the therapy, and
b) comparing the expression level of the CTA gene in the biological samples, wherein the therapy administered comprises a CaSR activator agent and wherein an increase of the expression level of the CTA gene after the therapy with respect to the expression level of said CTA gene before the therapy, is indicative that the therapy is effective.

18. Method according to claim 17, wherein the expression level is quantifying by any one method selected from:
a) detecting mRNA of the CTA gene;
b) detecting the protein encoded by the CTA gene; and
c) detecting any amino acid sequence that has at least 90% identity with the protein encoded by the CTA gene.

19. Method according to claim 17 or 18, wherein the biological sample is a tumor tissue sample or a biological fluid, preferably a biological fluid.

20. Method according to any one of claims 17-19, wherein the Ca-SR activator agent is a CaSR allosteric activator; preferably is selected from Cinacalcet, NPS-R568, calindol, calcimimetic B, AC-265347 and combinations thereof; and more preferably is Cinacalcet.

21. Method according to any one of claims 17-20, wherein the cancer is selected from parathyroid adenoma and carcinoma, colon cancer, breast cancer, prostate cancer, glioma, Leydig cell cancer, ovarian cancer, neuroblastomas, ganglioneuroblastomas and ganglioneuromas, bone metastases and combinations thereof; preferably selected from neuroblastomas, ganglioneuroblastomas and ganglioneuromas; and more preferably is neuroblastoma.

22. Method according to any one of claims 17-21, wherein step a) comprises quantifying the expression level of:
- a CTA of the SSX family; preferably SSX 4 and/or SSX 4B, and/or
- a CTA of the GAGE family; preferably selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof.

23. Method according to claim 22, wherein step a) comprises quantifying the expression level of a CTA of the MAGE family; preferably MAGE A2 and/or MAGE A3.

24. Method according to claim 22 or 23, wherein step a) comprises quantifying the expression level of a non-X-CTA; preferably CTCFL.

25. Kit to carry out the method as defined in any one of the claims 22-24, comprising appropriate means to quantify the expression level of:
- a CTA of the SSX family; preferably SSX 4 and/or SSX 4B, and
- a CTA of the GAGE family; preferably selected from GAGE1, GAGE 2A, GAGE 2B, GAGE 2C, GAGE 2D, GAGE 2E, GAGE 4, GAGE 5, GAGE 6, GAGE 7, GAGE 8, GAGE 10, GAGE 12B, GAGE 12C, GAGE 12D, GAGE 12E, GAGE 12F, GAGE 12G, GAGE 12H, GAGE 12I, GAGE 12J, GAGE 13 and combinations thereof,
and optionally:
- a CTA of the MAGE family; preferably MAGE A2 and/or MAGE A3, and/or
- a non-X-CTA; preferably CTCFL,
and a suitable packaging.

26. Use of a CTA gene as surrogate marker of tumor response of a CaSR-expressing cancer to treatment with a CaSR activator agent.

27. Use according to claim 26, wherein the CaSR activator agent is a CaSR allosteric activator, preferably is selected from Cinacalcet, NPS-R568, calindol, calcimimetic B, AC-265347 and combinations thereof, and more preferably is Cinacalcet.

28. Use according to claim 26 or 27, wherein the cancer is selected from parathyroid adenoma and carcinoma, colon cancer, breast cancer, prostate cancer, glioma, leydig cell cancer, ovarian cancer, neuroblastomas, ganglioneuroblastomas and ganglioneuromas, bone metastases and combinations thereof; preferably selected from neuroblastomas, ganglioneuroblastomas and ganglioneuromas; and more preferably is neuroblastoma.


SEQUENCE LISTING

<110>  HOSPITAL SANT JOAN DE DEU
       FUNDACIÓ CELLEX

<120>  INMUNOTERAPIA PARA EL CANCER

<130>  PCT20150380

<160>  20

<170>  PatentIn version 3.5

<210>  1
<211>  20
<212>  DNA
<213>  Artificial Sequence

<220>
<223>  OLIGONUCLEOTIDO SENTIDO CTCFL

<400>  1
gcacggaaaa gcgacctacg                                                   20


<210>  2
<211>  20
<212>  DNA
<213>  Artificial Sequence

<220>
<223>  OLIGONUCLEOTIDO ANTISENTIDO CTCFL

<400>  2
tgagggcata gcgttcatgg                                                   20


<210>  3
<211>  20
<212>  DNA
<213>  Artificial Sequence

<220>
<223>  OLIGONUCLEÓTIDO SENTIDO SSX4/4B

<400>  3
caccctccca cctttcatgc                                                   20


<210>  4
<211>  23
<212>  DNA
<213>  Artificial Sequence

<220>
<223>  OLIGONUCLEÓTIDO ANTISENTIDO SSX4/4B

<400>  4
ggacgttcaa cctgattcct gtg                                               23


<210>  5
<211>  20

<212>  DNA
<213>  Artificial Sequence

<220>
<223>  OLIGONUCLEÓTIDO SENTIDO GAGE-P1

<400>  5
gcagctgctc aggagggaga                                                    20


<210>  6
<211>  20
<212>  DNA
<213>  Artificial Sequence

<220>
<223>  OLIGONUCLEÓTIDO ANTISENTIDO GAGE-P1

<400>  6
tgtgggtgac cctgttcctg                                                    20


<210>  7
<211>  20
<212>  DNA
<213>  Artificial Sequence

<220>
<223>  OLIGONUCLEOTIDO SENTIDO GAGE-P2

<400>  7
ggccgaagcc tgaagctcat                                                    20


<210>  8
<211>  20
<212>  DNA
<213>  Artificial Sequence

<220>
<223>  OLIGONUCLEOTIDO ANTISENTIDO GAGE-P2

<400>  8
atttggcggg tccatctcct                                                    20


<210>  9
<211>  21
<212>  DNA
<213>  Artificial Sequence

<220>
<223>  OLIGONUCLEOTIDO SENTIDO GAGE-P3

<400>  9
gagggagcat ctgcaggtca a                                                  21


<210>  10
<211>  20
<212>  DNA
<213>  Artificial Sequence

<220>
<223>  OLIGONUCLEOTIDO ANTISENTIDO GAGE-P3

<400>  10
cctgcccatc aggaccatct                                                    20


<210>  11
<211>  20
<212>  DNA
<213>  Artificial Sequence

<220>
<223>  OLIGONUCLEOTIDO SENTIDO MAGE A2

<400>  11
aaccaggcag tgaggccttg                                                    20


<210>  12
<211>  20
<212>  DNA
<213>  Artificial Sequence

<220>
<223>  OLIGONUCLEOTIDO ANTISENTIDO MAGE A2

<400>  12
agcctgtccc cctcagaacc                                                    20


<210>  13
<211>  20
<212>  DNA
<213>  Artificial Sequence

<220>
<223>  OLIGONUCLEOTIDO SENTIDO MAGE A3

<400>  13
aaggtggccg agttggttca                                                    20


<210>  14
<211>  20
<212>  DNA
<213>  Artificial Sequence

<220>
<223>  OLIGONUCLEOTIDO ANTISENTIDO MAGE A3

<400>  14
actgccaatt tccgacgaca                                                    20


<210>  15
<211>  20
<212>  DNA
<213>  Artificial Sequence

<220>
<223>  OLIGONUCLEOTIDO SENTIDO ADCY8

<400>  15
cgccaagctt tcctggagac                                                   20


<210>  16
<211>  20
<212>  DNA
<213>  Artificial Sequence

<220>
<223>  OLIGONUCLEOTIDO ANTISENTIDO ADCY8

<400>  16
acgagccgct cctgtctttg                                                   20


<210>  17
<211>  20
<212>  DNA
<213>  Artificial Sequence

<220>
<223>  OLIGONUCLEOTIDO SENTIDO PRKCA

<400>  17
ctgagaagag ggggcggatt                                                   20


<210>  18
<211>  20
<212>  DNA
<213>  Artificial Sequence

<220>
<223>  OLIGONUCLEOTIDO ANTISENTIDO PRKCA

<400>  18
ggatctgaaa gcccgtttgg                                                   20


<210>  19
<211>  20
<212>  DNA
<213>  Artificial Sequence

<220>
<223>  OLIGONUCLEOTIDO SENTIDO RYR2

<400>  19
gctcaaggtg gtggtcatcg                                                   20


<210>  20
<211>  20
<212>  DNA
<213>  Artificial Sequence

<220>
<223>  OLIGONUCLEOTIDO ANTISENTIDO RYR2

<400>  20
ttgaagaccg ggaggtggac                                                   20